# EUROPEAN PATENT SPECIFICATION

(11) **EP 4 507 783 B1**
(45) Date of publication and mention of the grant of the patent: **25.11.2026**
(21) Application number: 23720241.1
(22) Date of filing: 12.04.2023
(51) Int. Cl.: A61P 11/06, G01N 33/68

(54) **BIOMARKERS FOR DIAGNOSING EQUINE ASTHMA**
BIOMARKER ZUM DIAGNOSTIZIEREN VON PFERDEASTHMA
BIOMARQUEURS POUR LE DIAGNOSTIC DE L'ASTHME ÉQUIN

(30) Priority: 13.04.2022 EP 22168093
(43) Date of publication of application: 19.02.2025
(73) Proprietor: Freie Universität Berlin, 14195 Berlin (DE)
(72) Inventor: MUNDHENK, Lars, 16515 Oranienburg (DE); BARTENSCHLAGER, Florian, 12163 Berlin (DE); GRUBER, Achim D., 14513 Teltow (DE); GEHLEN, Heidrun, 14532 Kleinmachnow (DE); WEISE, Christoph, 10627 Berlin (DE); KUROPKA, Benno, 10629 Berlin (DE); DUMKE, Fiona Lesanne, 15711 Königs Wusterhausen (DE)
(74) Representative: Maikowski & Ninnemann Patentanwälte Partnerschaft mbB
(86) International application number: PCT/EP2023/059550
(87) International publication number: WO 2023/198770

(56) References cited:
- GY CLEMENTINE ET AL: "Investigation of blood biomarkers for the diagnosis of mild to moderate asthma in horses", vol. 33, no. 4, 1 July 2019 (2019-07-01), US, pages 1789 - 1795, XP055955994, ISSN: 0891-6640, Retrieved from the Internet <URL:https://www.ncbi.nlm.nih.gov/pmc/articles/PMC6639487/pdf/JVIM-33-1789.pdf> DOI: 10.1111/jvim.15505
- RYHNER T ET AL: "Increased mucus accumulation in horses chronically affected with recurrent airway obstruction is not associated with up-regulation of CLCA1, EGFR, MUC5AC, Bcl-2, IL-13 and INF-@c expression", VETERINARY IMMUNOLOGY AND IMMUNOPATHOLOGY, ELSEVIER, AMSTERDAM, NL, vol. 125, no. 1-2, 15 September 2008 (2008-09-15), pages 8 - 17, XP023902221, ISSN: 0165-2427, [retrieved on 20080523], DOI: 10.1016/J.VETIMM.2008.05.011
- CHIARADIA ELISABETTA ET AL: "In slow pace towards the proteome of equine body fluids", JOURNAL OF PROTEOMICS, ELSEVIER, AMSTERDAM, NL, vol. 225, 19 June 2020 (2020-06-19), XP086219897, ISSN: 1874-3919, [retrieved on 20200619], DOI: 10.1016/J.JPROT.2020.103880
- GERBER V ET AL: "Mucin genes in horse airways: MUC5AC, but not MUC2, may play a role in recurrent airway obstruction", EQUINE VETERINARY JOURNAL, R & W PUBLICATIONS, SUFFOLK, GB, vol. 35, no. 3, 5 January 2010 (2010-01-05), pages 252 - 257, XP071653247, ISSN: 0425-1644, DOI: 10.2746/042516403776148291
- JEFCOAT A. M. ET AL: "Persistent mucin glycoprotein alterations in equine recurrent airway obstruction", vol. 281, no. 3, 1 September 2001 (2001-09-01), US, pages 704 - 712, XP055955962, ISSN: 1040-0605, Retrieved from the Internet <URL:https://journals.physiology.org/doi/pdf/10.1152/ajplung.2001.281.3.l704> DOI: 10.1152/ajplung.2001.281.3.L704
- BOXALL C ET AL: "THE CONTRIBUTION OF TRANSFORMING GROWTH FACTOR-BETA AND EPIDERMAL GROWTH FACTOR SIGNALLING TO AIRWAY REMODELLING IN CHRONIC ASTHMA", EUROPEAN RESPIRATORY JOURNAL, EUROPEAN RESPIRATORY SOCIETY, GB, vol. 27, no. 1, 1 January 2006 (2006-01-01), pages 208 - 229, XP009063244, ISSN: 0903-1936, DOI: 10.1183/09031936.06.00130004
- COUËTIL L.L. ET AL: "Inflammatory Airway Disease of Horses-Revised Consensus Statement", vol. 30, no. 2, 24 January 2016 (2016-01-24), US, pages 503 - 515, XP055956351, ISSN: 0891-6640, Retrieved from the Internet <URL:https://onlinelibrary.wiley.com/doi/full-xml/10.1111/jvim.13824> DOI: 10.1111/jvim.13824

## Description

The present invention relates to the in vitro use of mucin-4 as biomarker for diagnosing equine asthma according to the preamble of claim 1, to the in vivo diagnostic use of mucin-4 as biomarker for equine asthma according to the preamble of claim 6, and to a method for analyzing an isolated airway secretion sample of an equine patient in vitro according to the preamble of claim 7.

Equine asthma (EA) is a very common and significant inflammatory disease of the equine lung, which leads to, among other things, a massive reduction in performance, severe health damage and significant impairment of the general condition and usability of horses⁰. A main clinical and pathological feature is an overproduction of mucus (phlegm), which obstructs and constricts the airways and contributes to the restriction of respiratory function. There are mild to moderate and severe forms of equine asthma. Diagnosis of EA is currently associated with high interventional, financial, and time costs, due in part to a lack of known, easily collected, disease-specific parameters (biomarkers). The development of improved minimally invasive diagnostics that can be easily applied directly to the patient by practicing veterinarians or outright by patient owners is one of the most important challenges in equine medicine.

Chiaradia and Miller is a review on horse body fluid proteome analysis.⁶ Amongst others, the review discusses a publication in which overall 1003 proteins were identified from horse bronchoalveolar lavage fluid, wherein 108 of these proteins resulted to be significantly differentially abundant in asthmatics compared to healthy individuals. The authors of that publication focused their attention on four identified proteins, namely, on serotransferrin precursor, polymeric immunoglobulin receptor, surfactant protein A and cytochrome C. Chiaradia and Miller concluded that the reported findings reveal that further proteomic studies on equine body fluids collected from diseased animals are required.

It is an object of the present invention to provide a biomarker that is appropriate in diagnosing equine asthma.

This object is achieved with the use of mucin-4 (Muc4) as biomarker in an in vitro method for diagnosing equine asthma of an equine patient. Upon analyzing samples of healthy horses and horses suffering from equine asthma, the inventors identified mucin-4, polymeric immunoglobulin receptor (PIGR), carcinoembryonic antigen-related cell adhesion molecule 1 (CAECAM-1), CD177, and vanin 1 (VNN1) to be of particular relevance for distinguishing between healthy horses and horses suffering from equine asthma. Surprisingly, all of these proteins are membrane-linked glycoproteins. While it is not fully understood why membrane-linked glycoproteins are particularly appropriate to distinguish between horses suffering from equine asthma and healthy horses, it appears that the common structural motif of the identified proteins (i.e., the fact that they are membrane-bound and carry a glycosylation) qualifies them to be used as biomarkers for diagnosing equine asthma in a particularly appropriate manner. This is a particularly surprising finding, since the inventors analyzed the concentration of approximately 1900 proteins in the airway secretions of healthy horses and horses suffering from equine asthma. Many of these proteins do not belong to the group of membrane-linked glycoproteins. However, the proteins that were found to be particularly appropriate biomarkers for diagnosing equine asthma are such membrane-linked glycoproteins.

In an embodiment, a biomarker set comprising at least one additional protein chosen from the group consisting of polymeric immunoglobulin receptor, carcinoembryonic antigen-related cell adhesion molecule 1, CD177, and vanin 1 is used. Such a biomarker set can even increase the accuracy and reliability of diagnosing equine asthma of a horse. In an embodiment, at least 3, in particular at least 4, in particular at least 5 proteins are contained in the biomarker set. In an embodiment, exactly 2, 3, 4, 5, or 6 of the proteins are comprised in the biomarker set.

Herewith disclosed but not forming part of the claimed invention is the isolated use of polymeric immunoglobulin receptor as biomarker for diagnosing equine asthma.

Herewith disclosed but not forming part of the claimed invention is the isolated use of carcinoembryonic antigen-related cell adhesion molecule 1 as biomarker for diagnosing equine asthma.

Herewith disclosed but not forming part of the claimed invention is the isolated use of CD177 as biomarker for diagnosing equine asthma.

Herewith disclosed but not forming part of the claimed invention is the isolated use of vanin 1 as biomarker for diagnosing equine asthma.

It should be noted that vanin 1 can be present in at least two different isoforms. The term "vanin 1" as used herein relates to any of these isoforms, in particular to the isoforms explicitly described herein below. To present an easy overview of the novel biomarkers of equine asthma, identification information and structural details are summarized in the following Table 1.

**Table 1: Identification information and structural details of the newly identified protein biomarkers for equine asthma.**

| **UniProt Identifier ^{*)}** | **Database entry (FASTA header)** | **Protein name** | **SEQ ID NO.** |
|---|---|---|---|
| A0A5F5PQU9 | tr\|A0A5F5PQU9\|A0A5F5PQU9_HORSE Mucin-4 OS=Equus caballus OX=9796 GN=MUC4 PE=4 SV=1 caballus OX=9796 GN=MUC4 PE=4 SV=1 | Mucin-4 (Muc4) | 1 |
| F6W2K5 | tr\|F6W2K5\|F6W2K5_HORSE Polymeric immunoglobulin receptor OS=Equus caballus OX=9796 GN=PIGR PE=4 SV=2 | Polymeric immunoglobulin receptor (PIGR) | 2 |
| F6T1X0 | tr\|F6T1X0\|F6T1X0_HORSE Carcinoembryonic antigen-related cell adhesion molecule 1 OS=Equus caballus OX=9796 GN=LOC100069287 PE=4 SV=3 | Carcinoembryonic antigen-related cell adhesion molecule 1 (CAECAM-1) | 3 |
| F7C456 | tr\|F7C456\|F7C456_HORSE CD177 antigen OS=Equus caballus OX=9796 GN=LOC100069985 PE=4 SV=2 | CD177 | 4 |
| A0A3Q2I1X0 | tr\|A0A3Q2I1X0\|A0A3Q2I1X0_HORSE Vanin 1 OS=Equus caballus OX=9796 GN=VNN1 PE=3 SV=1 | Vanin 1 (VNN1) isoform 1 | 5 |
| A0A5F5PVM4 | tr\|A0A5F5PVM4\|A0A5F5PVM4_HORSE Vanin 1 OS=Equus caballus OX=9796 GN=VNN1 PE=3 SV=1 | Vanin 1 (VNN1) isoform 2 | 6 |

| | | | |
|---|---|---|---|
| ^{*)} The UniProt identifier allows accessing all relevant data on the respective protein via the database http://www.uniprot.org. | | | |

In an embodiment, the equine asthma is mild equine asthma. Mild equine asthma is characterized by mild symptoms such as moderate exercise intolerance or occasional cough. An absence of resting dyspnea, even when exposed to hay or straw, is a differentiating criterion of mild equine asthma from severe equine asthma. Horses suffering from mild equine asthma typically show no signs of systemic infection clinically or in blood work. The endoscopic picture is regularly characterized by moderately increased secretion volume and consistency (≥ 2/5 in the Thoroughbred or ≥ 3/5 in the Warmblood according to a scoring system reported previously¹). Cytologically, an increase in the percentage of neutrophils of more than 10 %, of eosinophils of more than 5 %, and/or of metachromatic cells of more than 5 % (in each case with respect to healthy horses) can be typically observed in bronchoalveolar lavage fluids of horses suffering from mild equine asthma. The results of simple forms of pulmonary functional diagnostics such as arterial blood gas analysis is typically unchanged with respect to healthy horses.

In an embodiment, the equine asthma is severe equine asthma. Severe equine asthma is characterized by expiratory or mixed resting dyspnea with increased respiratory rate, abdominal respiratory type, double-beat breathing, anal pumping, and possibly the formation of a heave line. As with mild equine asthma, affected horses typically fail to show signs of systemic infection clinically or on blood work. The endoscopic picture is regularly characterized by an often significantly increased amount and viscosity of secretions. Bronchoalveolar lavage fluids of horses suffering from severe equine asthma typically show a marked neutrophilia (more than 25 % increase in neutrophils with respect to healthy horses).

In an embodiment, the vanin 1 is a vanin 1 isoform having a primary sequence according to sequence ID number 5 (vanin 1 isoform 1).

In an embodiment, the vanin 1 is a vanin 1 isoform having a primary sequence according to sequence ID number 6 (vanin 1 isoform 2).

In an embodiment, both previously mentioned vanin 1 isoforms are combined with each other together with mucin-4 and optionally other proteins to make up the biomarker set.

In an embodiment, the biomarker set comprises or essentially consists of the following proteins: mucin-4 and polymeric immunoglobulin receptor.

In an embodiment, the biomarker set comprises or essentially consists of the following proteins: mucin-4 and carcinoembryonic antigen-related cell adhesion molecule 1.

In an embodiment, the biomarker set comprises or essentially consists of the following proteins: mucin-4, polymeric immunoglobulin receptor, and carcinoembryonic antigen-related cell adhesion molecule 1.

In an embodiment, the biomarker set comprises or essentially consists of the following proteins: mucin-4, polymeric immunoglobulin receptor, carcinoembryonic antigen-related cell adhesion molecule 1, and CD177.

In an embodiment, the biomarker set comprises or essentially consists of the following proteins: mucin-4, polymeric immunoglobulin receptor, carcinoembryonic antigen-related cell adhesion molecule 1, CD177, and vanin 1.

In an embodiment, the biomarker set comprises or essentially consists of the following proteins: mucin-4, polymeric immunoglobulin receptor, carcinoembryonic antigen-related cell adhesion molecule 1, and vanin 1.

In an embodiment, the biomarker set comprises or essentially consists of the following proteins: mucin-4, polymeric immunoglobulin receptor, CD177, and vanin 1.

In an embodiment, the biomarker set comprises or essentially consists of the following proteins: mucin-4, carcinoembryonic antigen-related cell adhesion molecule 1, CD177, and vanin 1.

In an embodiment, the biomarker set comprises or essentially consists of the following proteins: mucin-4, CD177, and vanin 1.

In an embodiment, the biomarker set comprises or essentially consists of the following proteins: mucin-4 and CD177.

In an embodiment, the biomarker set comprises or essentially consists of the following proteins: mucin-4 and vanin 1.

In an embodiment, the in vitro method is carried out by airway secretion analysis, in particular by mucus analysis. Airway secretions, in particular mucus, are easily available from horses without any surgical intervention.

In an aspect, the present invention relates to the medical use of mucin-4.

Herewith disclosed but not forming part of the claimed invention isa medical method of diagnosing equine asthma in an equine patient by analyzing an airway secretion sample of the equine patient with respect to the concentration of at least one protein chosen from the group of mucin-4, polymeric immunoglobulin receptor, carcinoembryonic antigen-related cell adhesion molecule 1, CD177, and vanin 1. This method comprises the steps explained in the following.

First, an airway secretion sample is taken from an equine patient.

Afterwards, the concentration of at least one protein chosen from the group consisting of mucin-4, polymeric immunoglobulin receptor, carcinoembryonic antigen-related cell adhesion molecule 1, CD177, and vanin 1 is determined in the isolated airway secretion sample. In an embodiment, this is done automatically and/or with the use of a computer.

Finally, it is determined that the equine patient suffers from equine asthma if the concentration of the at least one protein exceeds a predetermined threshold. In an embodiment, this is done automatically and/or with the use of a computer. This method can also be denoted as ex vivo method.

In an embodiment, the concentration of the at least one protein and/or the result indicating whether the equine patient suffers from equine asthma is output by a computer or a measuring device to a person (such as a veterinarian or an owner of the equine patient), e.g., in form of a test report.

In an aspect, the present invention relates to a method for analyzing an isolated airway secretion sample of an equine patient in vitro. This method comprises the steps explained in the following.

First, an isolated airway secretion sample originating from an equine patient is provided.

Afterwards, the concentration of at least mucin-4 is determined in the isolated airway secretion sample. This is done with an appropriate measuring technique typically requiring a measuring device. Particularly appropriate measuring techniques are spectroscopy (such as NMR spectroscopy or mass spectrometry), chromatography (such as liquid chromatography, in particular high-performance liquid chromatography (HPLC)) and antibody reactions resulting in a detectable change in color or fluorescence.

Finally, it is determined that the equine patient suffers from equine asthma if the concentration of mucin-4 exceeds a predetermined threshold.

It should be noted that the concentration is not necessarily an absolute concentration. Rather, the method can also be performed by determining a relative concentration of mucin-4. Such relative concentration is a semi-quantitative measure of the concentration of mucin-4, e.g., with respect to the concentration of the same protein in a healthy individual.

By relying on relative concentrations, any influences of sample obtaining and sample measuring are eliminated if a sample from an equine patient suspected of suffering from equine asthma is obtained and measured in the same way as a control sample of a healthy equine individual. The relative concentration can also be determined with respect to a control protein being present in the airway secretion of an equine patient suspected of suffering from equine asthma, but the concentration of which is essentially not affected by this disease. Then, only a single airway secretion sample needs to be taken from the equine patient without having the need of obtaining an additional sample from a healthy individual.

In an embodiment, the concentration is an absolute concentration indicating the concentration of mucin-4 in SI units (or any comparable units).

In an embodiment, the isolated airway secretion sample comprises respiratory mucus. Nasal mucus, tracheal mucus, tracheobronchial mucus, and bronchoalveolar mucus are particularly appropriate examples of respiratory mucus.

In an embodiment, the isolated airway secretion sample is a (complete) bronchoalveolar lavage fluid sample.

In an embodiment, the isolated airway secretion sample is a mucus plug. This mucus plug is obtained by centrifuging a bronchoalveolar lavage (BAL) fluid sample from an equine patient and gathering a pellet of the centrifuged bronchoalveolar lavage fluid sample. The supernatant can typically be discarded since it will be no longer needed if the analysis is performed on the mucus plug.

It turned out that the biomarkers according to the present disclosure are not only detectable in mucus or mucus-rich fractions of an airway secretion, but also in mucus-depleted or even mucus-free fractions. In an embodiment, the isolated airway secretion sample is a mucus-depleted sample.

In an embodiment, the isolated airway secretion sample is a mucus-free sample.

In an embodiment, the isolated airway secretion sample is a liquid obtained by centrifuging a bronchoalveolar lavage fluid sample from an equine patient and gathering a supernatant of the centrifuged bronchoalveolar lavage fluid sample. The supernatant is a mucus-depleted or mucus-free liquid. The pellet can typically be discarded since it will be no longer needed if the analysis is performed on the supernatant.

In an embodiment, the airway secretion sample is subjected to an ultrasonic treatment prior to determining the concentration of mucin-4. Such ultrasonic treatment guarantees for a particularly homogeneous distribution of the individual components of the airway secretion sample.

In an embodiment, the concentration of mucin-4 is determined by liquid chromatography-mass spectrometry (LC-MS). LC-MS is a particularly appropriate method for determining relative concentrations of proteins within the airway secretion sample.

In an embodiment, the concentration of mucin-4 is determined by subjecting the protein-containing sample to an antibody against mucin-4, wherein a binding between the antibody and the mucin-4 gives a detectable result, such as in case of a color or fluorescence reaction. Then, the detected color or fluorescence intensity can be used as measure for the concentration of mucin-4 in the airway secretion sample.

In an embodiment, the antibody is bound to a matrix. Then, the concentration of mucin-4 can be determined in a similar manner like the concentration of a virus (such as SARS-CoV-2), i.e., in a way very common to veterinary professionals and ordinary consumers due to extensive training over the last years. Such an arrangement makes it particularly easy to identify the result of a protein detection test.

All embodiments of the different uses can be combined in any desired way and can be transferred either individually or in any arbitrary combination to the respective other use, and to the described methods. Likewise, all embodiments of the described methods can be combined in any desired way and can be transferred either individually or in any arbitrary combination to the different uses or the respective other method.

Further details of aspects of the present invention will be explained in the following making reference to exemplary embodiments and accompanying Figures. In the Figures:
- Figure 1: shows a plot indicating the relative concentration of mucin-4 in the mucus of healthy horses and in the mucus of horses suffering from equine asthma;
- Figure 2: shows a plot indicating the relative concentration of polymeric immunoglobulin receptor in the mucus of healthy horses and in the mucus of horses suffering from equine asthma;
- Figure 3: shows a plot indicating the relative concentration of carcinoembryonic antigen-related cell adhesion molecule 1 in the mucus of healthy horses and in the mucus of horses suffering from equine asthma;
- Figure 4: shows a plot indicating the relative concentration of CD177 in the mucus of healthy horses and in the mucus of horses suffering from equine asthma;
- Figure 5: shows a plot indicating the relative concentration of vanin 1 isoform 1 in the mucus of healthy horses and in the mucus of horses suffering from equine asthma; and
- Figure 6: shows a plot indicating the relative concentration of vanin 1 isoform 2 in the mucus of healthy horses and in the mucus of horses suffering from equine asthma;

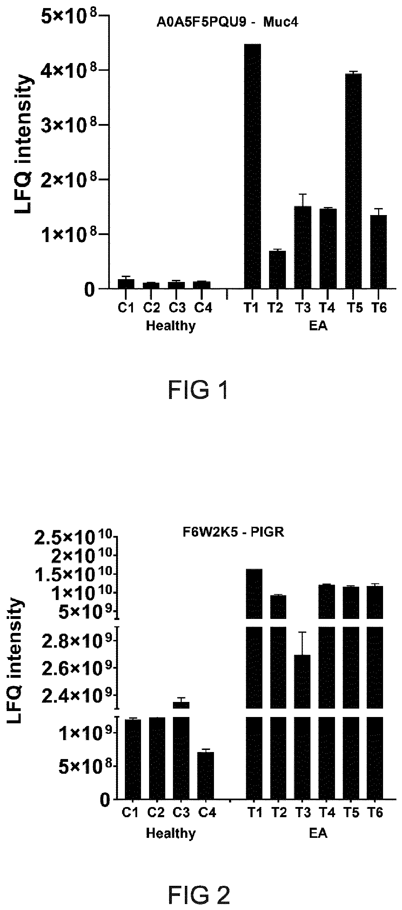

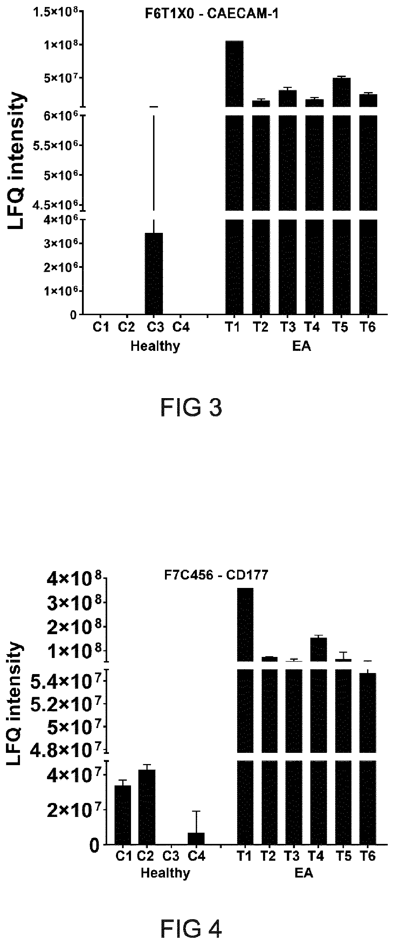

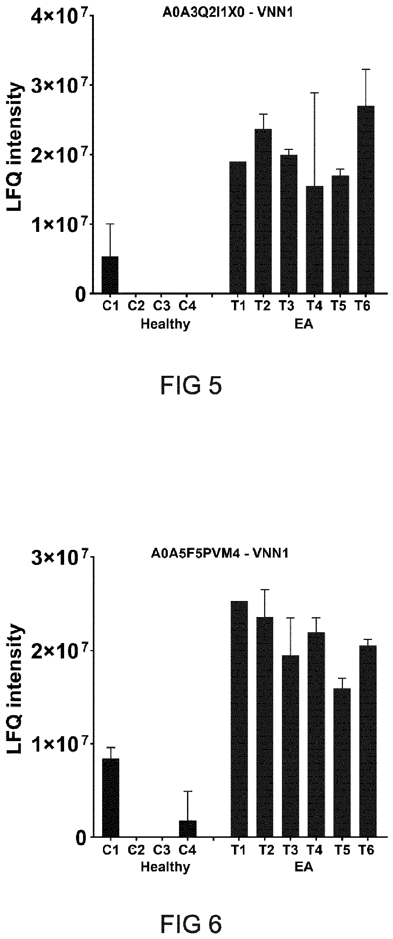

Figures 1 to 6 will be explained in the following in the context of an exemplary embodiment.

### Material and Methods

### BAL samples

Bronchoalveolar lavage (BAL) fluids were taken from horses which were clinically categorized in the following groups:
(1) lung healthy, n=4 and
(2) equine asthma (EA) (n=6).

The groups were defined as follows:
(1) healthy: no signs of lung disease;
(2) EA: The clinical picture had been present for more than 6 weeks and was characterized by clinical signs such as exercise intolerance, increased percentage of neutrophils in the BAL or occasional cough. The horses of this group showed no signs of local or systemic infection neither clinically nor in blood work.

### Mucus protein isolation and digestion

Mucus plugs were isolated and processed using the Fernandez-Blanco et al.² protocol with major modifications. Each BAL sample was centrifuged at room temperature (RT) at 300 x g for 30 min to remove cells. 5 % v/v of a saturated alcian blue 8GX in phosphate-buffered saline (PBS) solution was added to each supernatant and incubated over night at RT on a rotator (Thermo Fisher Scientific, Massachusetts, USA) at 40 revolutions/min (rpm).

The samples were subsequently centrifuged at RT at 14,000 x g for 30 min. The supernatant was removed and the pellet (in the following referred to as "mucus plug") was stored at -80 °C. The thawed mucus plug was resolubilized in reduction buffer (6 M guanidine hydrochloride; 0.1 M Tris-HCl, pH 8.5; 5 mM ethylenediaminetetraacetic acid; 0.1 M dithiothreitol; in ultra-pure water). Resolubilized mucus plug suspension (RPS) was squeezed four times through a 27 G cannula and subsequently incubated in an ultra-sonic bath for 20 min. The RPS samples were incubated at 37 °C overnight in a thermomixer at 500 rpm and dialyzed against FASP buffer A (0.1 M Tris-HCl in ultrapure water, pH 8.5) using the Slide-A-Lyzer^{™} MINI, 10K MWCO 2 ml dialysis unit (Thermo Fisher Scientific) according to the manufacturer's protocol.

Subsequently, protein concentrations were measured by fluorometry (Qubit^{™} Protein Assay Kit, Thermo Fisher Scientific) according to the manufacturer's protocol. 10 kDa cut-off filters (Nanosep Omega, Pall Corporation, New York, USA) were consecutively rinsed using 500 µl 70% methanol, 500 µl ultra-pure water, 500 µl 0.05 M sodium hydroxide in ultra-pure water, and two times 500 µl ultra-pure water. 45 µg total protein per RPS sample was subjected to filter-aided sample preparation (FASP). The samples were centrifuged at 14,000 x g for 10 min. The flow-through was discarded. The proteins on the filter were resuspended in 200 µl FASP buffer A and centrifuged at 14,000 x g for 40 min. The flow-through was discarded. The samples were incubated in 100 µl 0.05 M iodoacetamide (Promega, Wisconsin, USA) dissolved in FASP buffer A for 20 min in the dark. Samples were centrifuged at 14,000 x g for 30 min. Again, the flow- through was discarded.

The proteins on the filter were resuspended in 100 µl FASP buffer B (0.1 M Tris-HCl, pH 8.0) and centrifuged at 14,000 x g for 40 min. This procedure was repeated twice and the flow-throughs were again discarded. The proteins were pre-digested by resuspension in 40 µl FASP buffer B containing 1 µg endoproteinase Lys-C (Mass Spec Grade, Promega, Wisconsin, USA) on the filter for 4 h at 37 °C in a water bath. The filters were placed on a new collection tube, and 120 µl FASP buffer ABC (0.05 M ammonium bicarbonate in ultra-pure water) containing 1 µg trypsin (Trypsin Gold Mass Spec Grade, Promega) was added. The samples were incubated at 37 °C overnight in a water bath. Tryptic peptides were recovered by centrifugation at 14,000 x g for 20 min and transferred to a new 1.5 ml reaction tube. 50 µl of 0.5 M NaCl was added for elution of residual peptides and the flow-through was pooled with the first flow-through. Formic acid (LC-MS grade, Thermo Fisher Scientific) was added to a final concentration of 1 % to acidify the peptide solution. The acidified tryptic peptides were kept at -20°C. The peptides were desalted using stop-and-go-extraction tips (StageTips) prepared with C18 reversed-phase Empore disks (Merck Supelco, Mainz, Germany) as described³. Briefly, two layers of a C18 peptide desalting membrane (Thermo Fisher Scientific) were transferred to a 200 µl pipette tip (in the following referred to as "C18 tip"). The C18 tip was equilibrated by consecutive addition of the following solutions and centrifugation steps: 200 µl 100 % methanol at 700 x g for 1 min, 200 µl stage buffer B (50 % acetonitrile (Pierce^{™} Acetonitrile, LC-MS Grade, Thermo Fisher Scientific), 0.1 % formic acid (Thermo Fisher Scientific) in ultra-pure water) at 700 x g for 1 min, 200 µl stage buffer A (0.1 % formic acid (Thermo Fisher Scientific)in ultra-pure water) at 700 x g for 1.5 min. Finally, 200 µl stage buffer A was added and centrifuged at 700 x g for 2 min. The tryptic peptide solution was added to the C18 tip, centrifuged at 500 x g until the entire fluid phase passed the C18 membrane. Subsequently, 200 µl stage buffer A was added and centrifuged at 500 x g for 2 min. 100 µl stage buffer B was added and centrifuged at 500 x g for 5 min to elute the peptides. Eluted peptides were dried under vacuum. Three technical replicates were made for each sample. Only a single replicate was prepared for sample T1 as not enough protein was available.

### Quantitative proteome analysis by liquid chromatography-mass spectrometry (LC-MS)

Peptides were reconstituted in 15 µl of 0.05 % trifluoroacetic acid (TFA), 4 % acetonitrile, and 1 µl were analyzed by an Ultimate 3000 reversed-phase capillary nano-liquid chromatography system connected to a Q Exactive HF mass spectrometer (Thermo Fisher Scientific). Samples were injected and concentrated on a trap column (Acclaim PepMap100 C₁₈, 3 µm, 100 Å, 75 µm i.d. x 2 cm, Thermo Fisher Scientific) equilibrated with 0.05 % TFA in water. After switching the trap column inline, LC separations were performed on a capillary column (Acclaim PepMap100 C₁₈, 2 µm, 100 Å, 75 µm i.d. x 25 cm, Thermo Fisher Scientific) at an eluent flow rate of 300 nl/min. Mobile phase A contained 0.1 % formic acid in water, and mobile phase B contained 0.1 % formic acid in 80 % acetonitrile / 20 % water. The column was pre-equilibrated with 5 % mobile phase B followed by an increase of 5-44 % mobile phase B in 35 min. Mass spectra were acquired in a data-dependent mode utilising a single MS survey scan (m/z 350-1650) with a resolution of 60,000 in the Orbitrap, and MS/MS scans of the 15 most intense precursor ions with a resolution of 15,000. The dynamic exclusion time was set to 20 seconds and automatic gain control was set to 3x10⁶ and 1x10⁵ for MS and MS/MS scans, respectively. MS and MS/MS raw data were analyzed using the MaxQuant software package (version 2.0.1.0) with implemented Andromeda peptide search engine⁴.

Data were searched against the *Equus caballus* reference proteome downloaded from Uniprot (44,484 proteins, taxonomy 9796, last modified February 26, 2021) using the default parameters except for enabling the options label-free quantification (LFQ) and match between runs. Filtering and statistical analysis were performed using the software Perseus version 1.6.14 ⁵. Only proteins which were identified with LFQ intensity values in at least 6 replicates (within at least one of the pairwise comparisons of experimental groups) were used for downstream analysis. Missing values were replaced from normal distribution (imputation) using the default settings (width 0.3, down shift 1.8). Mean log2 fold protein LFQ intensity differences between experimental groups (EA vs. healthy) were calculated in Perseus using student's t-tests with false discovery rate-adjusted p-values of 0.05. Volcano plots were created by plotting the -log₁₀ p-value against the mean log₂ fold protein LFQ intensity differences. The LFQ data of the individual horses were also graphically illustrated using GraphPad Prism 6 (GraphPad Software Inc., La Jolla, USA).

### Results

Using a quantitative proteomics approach, a plurality of proteins was identified and quantified in mucus from horses with equine asthma compared to a healthy control group. Among the differentially expressed proteins the inventors discovered several membrane-linked glycoproteins in horses with equine asthma compared to a healthy control group that serve as biomarkers for equine asthma (Figures 1 to 6).

As can be seen from Figures 1 to 6, all individuals of the asthmatic group showed higher LFQ intensities of the indicated protein compared to the healthy horses. This qualifies the identified proteins as biomarkers for detecting (or diagnosing) equine asthma. The results illustrated in Figures 1 to 6 represent mean values with standard deviation of triplicates. The numbers refer to individual horses, wherein the letter "C" refers to control samples (healthy horses) and the letter "T" refers to test samples (asthmatic horses). If no bar is indicated, the respective protein concentration was beneath the detection limit.

### List of references cited in the preceding sections

0. Couetil, Laurent, et al. Equine asthma: current understanding and future directions. Frontiers in veterinary science 7 (2020): 450.
1. Gerber V, Straub R, Marti E, Hauptman J, Herholz C, King M, Imhof A, Tahon L, Robinson NE. Endoscopic scoring of mucus quantity and quality: observer and horse variance and relationship to inflammation, mucus viscoelasticity and volume. Equine Veterinary Journal. 2004;36(7):576-82.
2. Fernandez-Blanco JA, Fakih D, Arike L, Rodriguez-Pineiro AM, Martinez-Abad B, Skansebo E, Jackson S, Root J, Singh D, McCrae C, Evans CM, Astrand A, Ermund A, Hansson GC. Attached stratified mucus separates bacteria from the epithelial cells in COPD lungs. JCl Insight. 2018;3(17).
3. Rappsilber J, Mann M, Ishihama Y. Protocol for micro-purification, enrichment, pre-fractionation and storage of peptides for proteomics using StageTips. Nat Protoc. 2007;2(8):1896-906.
4. Tyanova S, Temu T, Cox J. The MaxQuant computational platform for mass spectrometry-based shotgun proteomics. Nat Protoc. 2016;11(12):2301-19.
5. Tyanova S, Temu T, Sinitcyn P, Carlson A, Hein MY, Geiger T, Mann M, Cox J. The Perseus computational platform for comprehensive analysis of (prote)omics data. Nat Methods. 2016;13(9):731-40.
6. Chiaradia, E., & Miller, I. (2020). In slow pace towards the proteome of equine body fluids. Journal of Proteomics, 2020; 225, 103880.

## Claims

1. Use of mucin-4 as biomarker in an in vitro method for diagnosing equine asthma of an equine patient.

2. Use according to claim 1, **characterized in that** a biomarker set comprising at least one additional protein chosen from the group consisting of polymeric immunoglobulin receptor, carcinoembryonic antigen-related cell adhesion molecule 1, CD177, and vanin 1 is used.

3. Use according to claim 2, **characterized in that** the vanin 1 is a vanin 1 isoform having a primary sequence according to SEQ ID NO. 5.

4. Use according to claim 2, **characterized in that** the vanin 1 is a vanin 1 isoform having a primary sequence according to SEQ ID NO. 6.

5. Use according to any of the preceding claims, **characterized in that** the in vitro method is carried out by airway secretion analysis.

6. Mucin-4 for use in in vivo diagnostics of equine asthma in an equine patient.

7. Method for analyzing an isolated airway secretion sample of an equine patient in vitro, the method comprising the following steps:
a) providing an isolated airway secretion sample originating from an equine patient,
b) determining the concentration of at least mucin-4 in the isolated airway secretion sample,
c) determining that the equine patent suffers from equine asthma if the concentration of mucin-4 exceeds a predeterminable threshold.

8. Method according to claim 7, **characterized in that** the isolated airway secretion sample comprises respiratory mucus.

9. Method according to claim 8, **characterized in that** the isolated airway secretion sample is a mucus plug obtained by centrifuging a bronchoalveolar lavage fluid sample from an equine patient and gathering a pellet of the centrifuged bronchoalveolar lavage fluid sample.

10. Method according to claim 7, **characterized in that** the isolated airway secretion sample is a mucus-depleted sample.

11. Method according to claim 7, **characterized in that** the isolated airway secretion sample is a mucus-free sample.

12. Method according to claim 10 or 11, **characterized in that** the isolated airway secretion sample is a liquid obtained by centrifuging a bronchoalveolar lavage fluid sample from an equine patient and gathering a supernatant of the centrifuged bronchoalveolar lavage fluid sample.

13. Method according to any of claims 7 to 12, **characterized in that** the isolated airway secretion sample is subjected to an ultrasonic treatment prior to determining the concentration of the at least one protein.

14. Method according to any of claims 7 to 13, **characterized in that** the concentration of the at least one protein is determined by liquid chromatography-mass spectrometry.

15. Method according to any of claims 7 to 13, **characterized in that** the concentration of the at least one protein is determined by an antibody-mediated color or fluorescence reaction.

## Patentansprüche

1. Verwendung von Mucin-4 als Biomarker in einem In-vitro-Verfahren zur Diagnose von Pferdeasthma bei einem Pferdepatienten.

2. Verwendung nach Anspruch 1, **dadurch gekennzeichnet, dass** ein Biomarker-Set verwendet wird, das mindestens ein zusätzliches Protein umfasst, das aus der aus polymerem Immunglobulinrezeptor, karzinoembryonalem Antigen-assoziiertem Zelladhäsionsmolekül 1, CD177 und Vanin 1 bestehenden Gruppe ausgewählt ist.

3. Verwendung nach Anspruch 2, **dadurch gekennzeichnet, dass** das Vanin 1 eine Vanin-1-Isoform mit einer Primärsequenz gemäß SEQ ID NO. 5. ist.

4. Verwendung nach Anspruch 2, **dadurch gekennzeichnet, dass** das Vanin 1 eine Vanin-1-Isoform mit einer Primärsequenz gemäß SEQ ID NO. 6 ist.

5. Verwendung nach einem der vorhergehenden Ansprüche, **dadurch gekennzeichnet, dass** das In-vitro-Verfahren mittels einer Analyse der Atemwegssekrete durchgeführt wird.

6. Mucin-4 zur Verwendung bei der In-vivo-Diagnostik von Pferdeasthma bei einem Pferdepatienten.

7. Verfahren zum Analysieren einer isolierten Probe von Atemwegssekreten bei einem Pferdepatienten in vitro, wobei das Verfahren die folgenden Schritte umfasst:
a) Bereitstellen einer isolierten Probe von Atemwegssekreten, die von einem Pferdepatienten stammt,
b) Ermitteln der Konzentration zumindest von Mucin-4 in der isolierten Probe von Atemwegssekreten,
c) Feststellen, dass der Pferdepatient an Pferdeasthma leidet, wenn die Konzentration von Mucin-4 einen vorab festlegbaren Schwellenwert übersteigt.

8. Verfahren nach Anspruch 7, **dadurch gekennzeichnet, dass** die isolierte Probe von Atemwegssekreten Atemwegsschleim umfasst.

9. Verfahren nach Anspruch 8, **dadurch gekennzeichnet, dass** die isolierte Probe von Atemwegssekreten ein Schleimpfropf ist, der durch Zentrifugieren einer bronchoalveolären Lavageflüssigkeitsprobe von einem Pferdepatienten und Entnehmen eines Pellet aus der zentrifugierten bronchoalveolären Lavageflüssigkeitsprobe gewonnen wird.

10. Verfahren nach Anspruch 7, **dadurch gekennzeichnet, dass** die isolierte Probe von Atemwegssekreten eine Probe mit weniger Schleim ist.

11. Verfahren nach Anspruch 7, **dadurch gekennzeichnet, dass** die isolierte Probe von Atemwegssekreten eine schleimfreie Probe ist.

12. Verfahren nach Anspruch 10 oder 11, **dadurch gekennzeichnet, dass** die isolierte Probe von Atemwegssekreten eine Flüssigkeit ist, die durch Zentrifugieren einer bronchoalveolären Lavageflüssigkeitsprobe von einem Pferdepatienten und Entnehmen eines Überstands der zentrifugierten bronchoalveolären Lavageflüssigkeitsprobe gewonnen wird.

13. Verfahren nach einem der Ansprüche 7 bis 12, **dadurch gekennzeichnet, dass** die isolierte Probe von Atemwegssekreten vor der Bestimmung der Konzentration des mindestens einen Proteins einer Ultraschallbehandlung unterzogen wird.

14. Verfahren nach einem der Ansprüche 7 bis 13, **dadurch gekennzeichnet, dass** die Konzentration des mindestens einen Proteins mittels Flüssigchromatographie-Massenspektrometrie bestimmt wird.

15. Verfahren nach einem der Ansprüche 7 bis 13, **dadurch gekennzeichnet, dass** die Konzentration des mindestens einen Proteins durch eine antikörpervermittelte Farb-oder Fluoreszenzreaktion bestimmt wird.

## Revendications

1. Utilisation de la mucine-4 comme biomarqueur dans une méthode in vitro pour le diagnostic de l'asthme équin chez un patient équin.

2. Utilisation selon la revendication 1, **caractérisée en ce qu'**on utilise un ensemble de biomarqueurs comprenant au moins une protéine supplémentaire choisie parmi le groupe constitué du récepteur polymérique des immunoglobulines, de la molécule d'adhésion cellulaire 1 liée à l'antigène carcino-embryonnaire, du CD177 et de la vanine 1.

3. Utilisation selon la revendication 2, **caractérisée en ce que** la vanine 1 est une isoforme de la vanine 1 présentant une séquence primaire conforme à la séquence SEQ ID NO. 5.

4. Utilisation selon la revendication 2, **caractérisée en ce que** la vanine 1 est une isoforme de la vanine 1 présentant une séquence primaire conforme à la séquence SEQ ID NO. 6.

5. Utilisation selon l'une quelconque des revendications précédentes, **caractérisée en ce que** la méthode in vitro est mise en œuvre par analyse de sécrétions des voies respiratoires.

6. La mucine-4 destinée à être utilisée dans le diagnostic in vivo de l'asthme équin chez un patient équin.

7. Procédé d'analyse in vitro d'un échantillon isolé de sécrétions des voies respiratoires d'un patient équin, ledit procédé comprenant les étapes suivantes :
a) fournir un échantillon isolé de sécrétions des voies respiratoires provenant d'un patient équin,
b) déterminer la concentration d'au moins la mucine-4 dans l'échantillon isolé de sécrétions des voies respiratoires,
c) déterminer que le patient équin est atteint d'asthme équin si la concentration en mucine-4 dépasse un seuil prédéterminable.

8. Procédé selon la revendication 7, **caractérisé en ce que** l'échantillon isolé de sécrétions des voies respiratoires comprend du mucus respiratoire.

9. Procédé selon la revendication 8, **caractérisé en ce que** l'échantillon isolé de sécrétions des voies respiratoires est un bouchon muqueux obtenu par centrifugation d'un échantillon de fluide de lavage broncho-alvéolaire provenant d'un patient équin et par récupération d'une pastille du fluide de lavage broncho-alvéolaire centrifugé.

10. Procédé selon la revendication 7, **caractérisé en ce que** l'échantillon isolé de sécrétions des voies respiratoires est un échantillon appauvri en mucus.

11. Procédé selon la revendication 7, **caractérisé en ce que** l'échantillon isolé de sécrétions des voies respiratoires est un échantillon exempt de mucus.

12. Procédé selon la revendication 10 ou 11, **caractérisé en ce que** l'échantillon isolé de sécrétions des voies respiratoires est un liquide obtenu par centrifugation d'un échantillon de fluide de lavage broncho-alvéolaire provenant d'un patient équin et par récupération d'un surnageant de cet échantillon de fluide de lavage broncho-alvéolaire centrifugé.

13. Procédé selon l'une quelconque des revendications 7 à 12, **caractérisé en ce que** l'échantillon isolé de sécrétions des voies respiratoires est soumis à un traitement par ultrasons avant la détermination de la concentration de ladite au moins une protéine.

14. Procédé selon l'une quelconque des revendications 7 à 13, **caractérisé en ce que** la concentration de ladite au moins une protéine est déterminée par chromatographie liquide couplée à la spectrométrie de masse.

15. Procédé selon l'une quelconque des revendications 7 à 13, **caractérisé en ce que** la concentration de ladite au moins une protéine est déterminée par une réaction colorée ou fluorescente médiée par un anticorps.
